# EUROPEAN PATENT APPLICATION

(11) **EP 1 201 234 A2**
(43) Date of publication of application: **02.05.2002**
(21) Application number: 00121508.6
(22) Date of filing: 29.09.2000
(51) Int. Cl.: A61K 31/13, A61P 43/00, A61P 35/02, A61P 27/16

(54) **Treatment of diseases with adamantane derivates**

(30) Priority: 21.09.2000 EP 00120660
(71) Applicant: Tinnitus Forschungs- und Entwicklungs GmbH, 80333 München (DE)
(72) Inventor: Ruppersberg, J. Peter, 72072 Tèbingen (DE); Fakler, Bernd, 72076 Tübingen (DE)
(74) Representative: Patentanwälte Ruff, Wilhelm, Beier, Dauster & Partner

(57) **Abstract**

The invention relates to the use of substances for the manufacturing of a pharmaceutical composition or medicament for the treatment of disturbances or illnesses which are linked to malfunction of an ionotropic acetylcholine receptor and/or a calcium-activated potassium channel functionally associated with said acetylcholine receptor, wherein said substance is at least one adamantane derivative.

## Description

The invention relates to the use of substances for the manufacturing of a pharmaceutical composition or medicament for the treatment of disturbances or illnesses which are linked to malfunction of an ionotropic acetylcholine receptor. Furthermore, the invention relates to the use of substances for the manufacturing of a pharmaceutical composition or a medicament for the treatment of disturbances or illnesses which are linked to malfunction of a calcium-activated potassium channel functionally associated with that ionotropic acetylcholine receptor.

The acetylcholine receptor is a ligand-gate cation channel. It is a glycoproteine composed of different types of trans-membraned polypeptides. Each of those polypeptides is coded by a separate gene, although they all show strong similarities in their amino acid sequences, implying that their genes have evolved from a single gene.

Like the voltage-gated Na⁺-channel, the acetylcholine-gated channel has a number of discrete alternative conformations and in the presence of the ligand jumps randomly from one to another, switching abruptly between closed and opened states. Once it has bound acetylcholine and made the transition to the opened stage, it remains open for a randomly variable length of time, averaging about one millisecond. Greatly prolongated exposure to acetylcholine causes the channel to enter a desensitized state. In the open conformation, the channel has a lumen at its extracellular end, narrowing towards cell interior to a small pore. The charge distribution in the channel wall is such that negative ions are excluded, while any positive ion can pass through. The normal traffic consists of Na⁺ and K⁺, together with some Ca²⁺. Since there is little selectivity among cations, their relative contributions to the current through the channel depends mainly on their concentrations and on the electrochemical driving forces. Opening of the acetylcholine receptor channel leads to a large net influx of positive ions, causing membran depolarization. This membran depolarization causes a signal transmission from one membran to another. A malfunction of this receptor can cause several diseases, and a malfunction of the acetycholine receptor in the brain is e.g. discussed as reason for Alzheimer disease.

It is the object of the invention to convert new scientific insights into a new strategy for the treatment of important diseases. This object is solved by the subject-matter of claims 1 and 8. Preferred embodiments are given in the dependent claims 2 to 7 and 9 to 15. The wording of all these claims is hereby incorporated into the content of the description by reference.

Surprisingly it was found, that the acetylcholine receptor in the inner ear comprises of two subunits, namely alpha9 and alpha10. These subunits can be blocked by memantine, an adamantane derivative known for treatment of tinnitus associated with a so-called positive recruitment and/or a reduction or failure of otoacoustic emissions (EP 0759 295).

Furthermore, it was found, that this acetycholine receptor in the inner ear is functionally associated with a calcium-activated potassium channel, namely of the SK subtype. These findings make it possible to use adamantane derivatives for the treatment of disturbances that are linked to malfunction of this acetylcholine receptor and/or this calcium-activated potassium channel.

According to the invention, at least one substance is used for the manufacturing of a pharmaceutical composition or a medicament for the treatment of disturbances or illnesses which are linked to malfunction of at least one ionotropic acetylcholine receptor, wherein that substance is an adamantane derivative and wherein that disturbance or illness is not a tinnitus associated with a so-called positive recruitment and/or a reduction or failure of otoacoustic emissions. According to the present invention, the acetylcholine receptor preferably comprises at least one so-called alpha9-subunit and alternatively or in addition it comprises at least one so-called alpha10-subunit. The substance which is used for the manufacturing of a pharmaceutical composition or medicament can be used in the form of its pharmaceutical acceptable salts and/or optionally together with a pharmaceutically carrier.

According to the invention, the above mentioned acetylcholine receptor is preferably functionally associated with at least one calcium-activated potassium channel. This calcium-activated potassium channel can be characterized in that it is of SK (small conductance) subtype, wherein preferably that SK potassium channel is of the SK2 subtype.

In one preferred embodiment of this invention the adamantane derivatives which are used for the manufacturing of a pharmaceutical composition or a medicament are of the formula 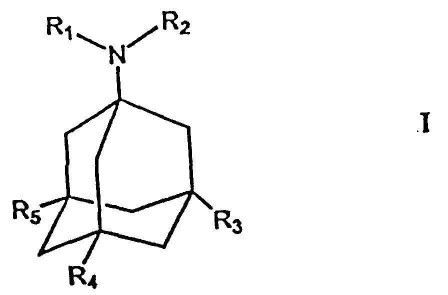 where R₁ and R₂
- are the same or different, and include hydrogen or straight or branched chain alkyl groups having 1 to 6 C atoms, or
- together with the N atom present a heterocyclic group having 5 or 6 ring atoms,
where R₃ and R₄ are the same or different, and include hydrogen, straight or branched chain alkyl groups having 1 to 6 C atoms, cycloalkyl groups having 5 to 6 C atoms, or phenyl, and
where R₅ is hydrogen or a straight or branched chain alkyl group having 1 to 6 C atoms.

In a preferred embodiment of this invention, this adamantane derivative is 3.5-dimethyl-1-adamantanamine or the hydrochloride thereof.

In other preferred embodiments according to the invention, the use of the substance is claimed for the manufacturing of a pharmaceutical composition or a medicament for the treatment of disturbances or illnesses linked to malfunction of at least one calcium-activated potassium channel functionally associated with a ionotropic acetylcholine receptor, wherein that substance is an adamantane derivative and wherein that disturbance or illness is not a tinnitus associated with a so-called positive recruitment and/or a reduction or failure of otoacoustic emissions.

According to the invention, this acetylcholine receptor which is functionally linked with a calcium-activated potassium channel preferably comprises at least one so-called alpha9-subunit and alternatively or in addition it comprises at least one so-called alpha10-subunit.

In a preferred embodiment according to the invention, the calcium-activated potassium channel is of the SK subtype, wherein preferably that SK potassium channel is of the SK2 subtype.

According to the invention, the adamantane derivative which can be used for the manufacturing of a pharmaceutical composition or a medicament for the treatment of disturbances or illnesses that are linked to malfunctions of the above mentioned potassium channel are of the formula 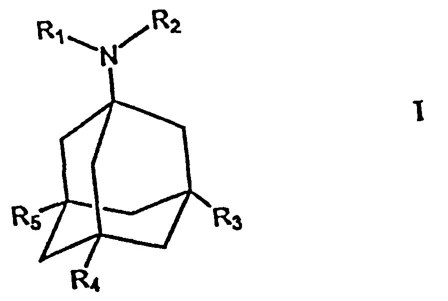 where R₁ and R₂
- are the same or different, and include hydrogen or straight or branched chain alkyl groups having 1 to 6 C atoms, or
- together with the N atom present a heterocyclic group having 5 or 6 ring atoms,
where R₃ and R₄ are the same or different, and include hydrogen, straight or branched chain alkyl groups having 1 to 6 C atoms, cycloalkyl groups having 5 to 6 C atoms, or phenyl, and
where R₅ is hydrogen or a straight or branched chain alkyl group having 1 to 6 C atoms.

Preferably this adamantane derivative is 3.5-dimethyl-1-adamantanamine or the hydrochloride thereof.

The invention can be useful for treatment of all kinds of disturbances or illnesses which are linked with malfunction of an ionotropic acetylcholine receptor as defined above or the calcium-activated potassium channel which is functionally associated with the above mentioned acetylcholine receptor and which is also defined above. One preferred disturbance which can be treated by the invention is leukaemia, but also other known or up to now unknown diseases which are linked to these malfunctions defined above can be treated.

Another preferred illness to be treated according the invention is deafness, especially inner ear deafness. It is known that outer hair cells comprise an acetylcholine receptor and that alpha9- and alpha10-subunits of this receptor are expressed in outer hair cells. As this receptor is permeable for Ca²⁺-ions, a toxic effect of such Ca²⁺-ions is possible, resulting in a temporary or permanent impairment of the outer hair cells. Such impairment can be amended or even cured by the inventive use. In a preferred embodiment according to the invention the inner ear deafness is a acute one, prior to the manifestation of a permanent state of inner ear deafness.

Finally, according to the invention it is possible to select the administration form of the substance. This form can be adapted to the age, sex or other characteristics of the patient, the severity of the disturbances or illnesses or other parameters. Conventional additives can be present. The dosage can be freely selected as a function of the clinical picture and the condition of the patient.

The described features and further features of the invention can be gathered from the following description of preferred embodiments in conjunction with the subclaims. The individual features can be implemented seperately or in the form of subcombinations.

### MATERIALS AND METHODS

### Electrophysiology on OHCs

Organs of Corti were dissected from the cochleae of Wistar rats (11-24 days old) as described previously (Oliver, D. and Fakler, B. 1999. J. Physiol. (Lond.) 519 pt. 3, 791-800; Oliver, D., Klöcker, N., Schluck, J., Baukrowitz, T., Ruppersberg, J.P. and Fakler, B. 2000. Neuron 26, 591-601). The electrophysiology on OHCs was also described previously (Oliver, D. et al., 2000, see above).

### Electrophysiology on heterologously expressed proteins

In vitro mRNA synthesis and oocyte injections were performed as previously described (Fakler, B., Brandle, U., Glowatzki, E., Weidemann, S., Zenner, H.P. and Ruppersberg, J.P. 1995. Cell 80, 149-154; Oliver, D. et al., 2000, see above). The electrophysiology on oocytes was also performed as described previously (Oliver, D. et al., 2000, see above).

### Figure legends:

Fig. 1: Memantine dose-responses determined for the inhibitory postsynaptic currents (IPSC) in outer hair cells (OHCs).

Fig. 2: Inhibition of alpha9/alpha10 heteromeric acetylcholine receptors expressed in Xenopus oocytes by memantine

### Experiment 1:

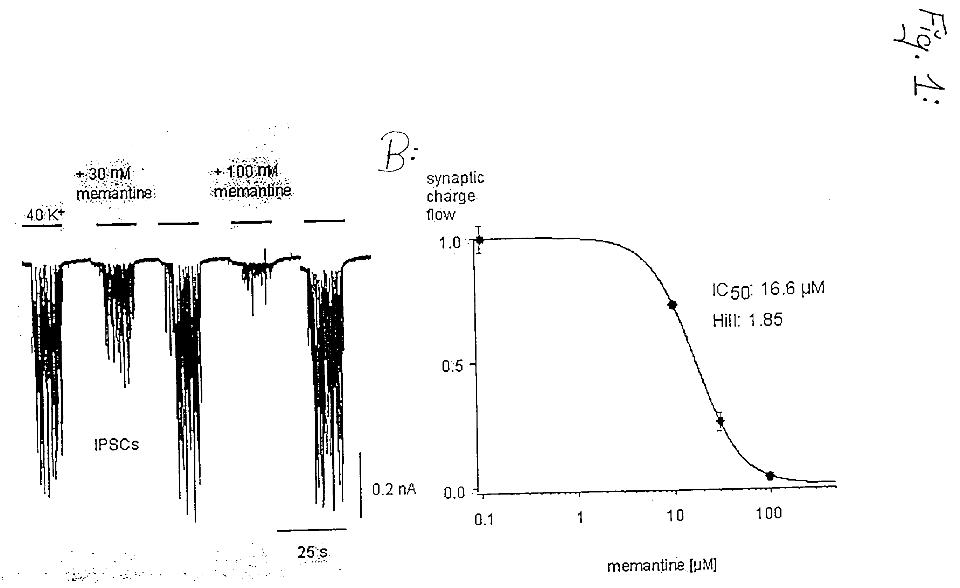

In Fig 1, postsynaptic currents were recorded from OHCs in voltage-clamp experiments when cells were held at -64 mV and the whole Corti preparation was superfused with high extracellular K⁺ to depolarize the presynapse. Application of high K⁺ and toxins as indicated by horizontal bars: current and time scalling as indicated. The postsynaptic currents were reversibly inhibited by application of memantine, whereas there is a dose-dependent response of the OHCs (30 mM and 100 mM, respectively). This dose-dependent response determined for the IPSC in OHCs is illustrated in Fig. 1B. There it is shown, that in OHCs memantine has an IC₅₀ of approximately 16.6 µM.

### Experiment 2:

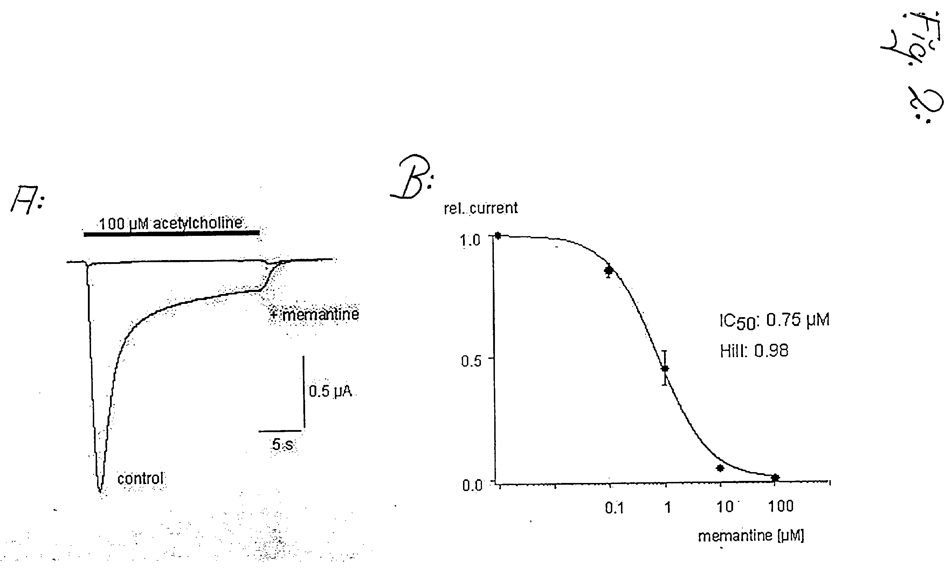

For further characterization of memantine treatment on the nAChR, the mRNA of alpha9/alpha10 subunits of the acetylcholine receptor were injected in oocytes of Xexopus laevis. After expression of that subunits, these subunits were investigated by treatment with memantine in voltage-clamp experiment. The cells were held at -30 mV. Current and time scaling are as indicated. As is shown in Fig. 2, similar results were obtained as in Fig. 1. In this experiment the polarization potential of the cells was meassured after application of 100 µM acetylcholine in the presence or absence of memantine. Consistent with the results obtained in Fig. 1, the current responses at the acetylcholine receptor could be blocked by application with memantine. A halfmaximal inhibition occurs at concentrations of approximately 1 µM, as is illustrated in Figur 2B.

## Claims

1. Use of a substance for the manufacturing of a pharmaceutical composition or medicament for the treatment of disturbances or illnesses linked to malfunction of at least one ionotropic acetylcholine receptor, wherein said substance is at least one adamantane derivative and wherein said disturbance or illness is not a tinnitus associated with a so-called positive recruitment and/or a reduction or failure of otoacoustic emissions.

2. Use according to claim 1, **characterized in that** said acetylcholine receptor comprises at least one alpha9-subunit.

3. Use according to claim 1 or claim 2, **characterized in that** said acetylcholine receptor comprises at least one alpha10-subunit.

4. Use according to one of the preceding claims, **characterized in that** said acetylcholine receptor is functionally associated with at least one calcium-activated potassium channel.

5. Use according to claim 4, **characterized in that** said calcium-activated potassium channel is of SK subtype, wherein preferably said SK potassium channel is SK2.

6. Use according to one of the preceding claims, **characterized in that** said adamantane derivatives are of the formula 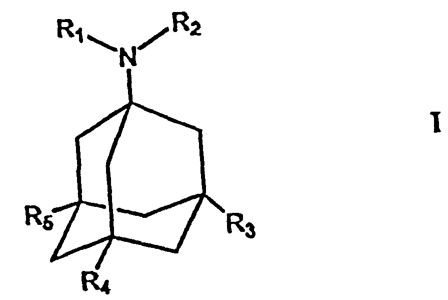 where R₁ and R₂
- are the same or different, and include hydrogen or straight or branched chain alkyl groups having 1 to 6 C atoms, or
- together with the N atom present a heterocyclic group having 5 or 6 ring atoms,
where R₃ and R₄ are the same or different, and include hydrogen, straight or branched chain alkyl groups having 1 to 6 C atoms, cycloalkyl groups having 5 to 6 C atoms, or phenyl, and
where R₅ is hydrogen or a straight or branched chain alkyl group having 1 to 6 C atoms.

7. Use according to claim 6, **characterized in that** said adamantane derivative is 3.5-dimethyl-1-adamantanamine or the hydrochloride thereof.

8. Use of a substance for the manufacturing of a pharmaceutical composition or a medicament for the treatment of disturbances or illnesses linked to malfunction of at least one calcium-activated potassium channel functionally associated with an ionotropic acetylcholine receptor, wherein said substance is at least one adamantane derivative and wherein said disturbance or illness is not a tinnitus associated with a so-called positive recruitment and/or a reduction or failure of otoacoustic emissions.

9. Use according to claim 8, **characterized in that** said acetylcholine receptor comprises at least one alpha9-subunit.

10. Use according to claim 8 or claim 9, **characterized in that** said acetylcholine receptor comprises at least one alpha10-subunit.

11. Use according to one of claims 8 to 10, **characterized in that** said calcium-activated potassium channel is of SK subtype, wherein preferably said SK potassium channel is SK2.

12. Use according to one of claims 8 to 11, **characterized in that** said adamantane derivatives are of the formula 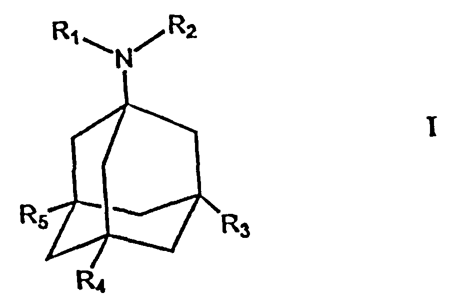 where R₁ and R₂
- are the same or different, and include hydrogen or straight or branched chain alkyl groups having 1 to 6 C atoms, or
- together with the N atom present a heterocyclic group having 5 or 6 ring atoms,
where R₃ and R₄ are the same or different, and include hydrogen, straight or branched chain alkyl groups having 1 to 6 C atoms, cycloalkyl groups having 5 to 6 C atoms, or phenyl, and
where R₅ is hydrogen or a straight or branched chain alkyl group having 1 to 6 C atoms.

13. Use according to claim 12, **characterized in that** said adamantane derivative is 3.5-dimethyl-adamantanamine or the hydrochloride thereof.

14. Use according to one of the preceding claims, **characterized in that** said disturbance or illness is leukaemia.

15. Use according to one of claims 1 to 13, **characterized in that** said disturbance or illness is deafness, especially inner ear deafness.
